(19) Europäisches Patentamt
European Patent Office
Office européen des brevets

(11) EP 0 737 691 B1

(12) **EUROPEAN PATENT SPECIFICATION**

(45) Date of publication and mention of the grant of the patent:
**09.09.1998 Bulletin 1998/37**

(51) Int Cl.[6]: **C07K 7/08**, A61K 38/08

(21) Application number: **95301627.6**

(22) Date of filing: **13.03.1995**

# (54) Bombesin analogs

Bombesin-Analogen

Analogues de bombésine

(84) Designated Contracting States:
**AT BE CH DE DK ES FR GB GR IE IT LI LU MC NL PT SE**
Designated Extension States:
**LT SI**

(43) Date of publication of application:
**16.10.1996 Bulletin 1996/42**

(73) Proprietor: **BIOMEASURE, INC.**
**Milford, Massachusetts 01757-2650 (US)**

(72) Inventors:
- **Kim, Sun Hyuk**
  **Massachusetts 02167 (US)**
- **Moreau, Jaques-Pierre**
  **Upton, Massachusetts 01568 (US)**

(74) Representative: **Armitage, Ian Michael et al**
**MEWBURN ELLIS**
**York House**
**23 Kingsway**
**London WC2B 6HP (GB)**

(56) References cited:
**WO-A-91/17181**

- **BIOCHEMISTRY., vol.29, no.3, 1990, EASTON, PA US pages 616 - 622 L H WANG ET AL. 'Desemthionine alkylamide bombesin analogues; a new class of bombesin receptor antagonists with potent secretory activity in pancratic acini and antimitotic activity in Swiss 3T3 cells'**

Remarks:
The file contains technical information submitted after the application was filed and not included in this specification

EP 0 737 691 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### BACKGROUND OF THE INVENTION

This invention relates to bombesin analogs useful for treatment of benign or malignant proliferation of tissue.

The amphibian peptide bombesin (see Anastasi, et al., Experientia 27:166-167, 1971) is closely related to the mammalian homolog gastrin-releasing peptide (GRP), neuromedin B (NMB), neuromedin C (NMC), and litorin. See Minamino, et al. Ann. N.Y. Acad. Sci. 547: 373-390 (1988). Both GRP and NMB receptors have been identified and characterized on human tumors. See Taylor, et al., Growth Factors, Peptides, and Receptors (ed. Moody, T. 1993). Bombesin has been found to be a growth factor for a number of human cancer cell lines, including small-cell lung carcinoma (SCLC), and has been detected in human breast and prostate cancer. Haveman, et al., eds. Recent Results in Cancer Research - Peptide Hormones in Lung Cancer, Springer-Verlag, New York, 1986. A number of these cancers are known to secrete peptide hormones related to GRP or bombesin. Consequently, antagonists to bombesin have been proposed as agents for the treatment of these cancers.

Cuttitta, et al. demonstrated that a specific monoclonal antibody to bombesin inhibited in vivo the growth of a human small-cell lung cancer cell line xenografted to nude mice. Cuttitta, et al., Cancer Survey 4:707-727, 1985. In 3T3 murine fibroblasts which are responsive to the mitotic effect of bombesin, Zachary and Rozengurt observed that a substance P antagonist, Spantide, acted as a bombesin antagonist. Zachary, et al., Proc. Natl. Acad. Sci. (USA), 82: 7616-7620, 1985. Heinz-Erian, et al. replaced His at position 12 in bombesin with D-Phe and observed bombesin antagonist activity in dispersed acini from guinea pig pancreas. Heinz-Erian, et al., Am. J. of Physiol. 252:G439-G442, 1987. Rivier reported work directed toward restricting the conformational freedom of the bioactive C-terminal decapeptide of bombesin by incorporating intramolecular disulfide bridges; however, Rivier mentioned that, so far, bombesin analogs with this modification fail to exhibit any antagonist activity. Rivier, et al., "Competitive Antagonists of Peptide Hormones," in Abstracts of the International Symposium on Bombesin-Like Peptides in Health and Disease, Rome, Italy October, 1987. Synthetic analogs of bombesin have also been reported in PCT Application WO 91/17181 (1991); Coy, et al. J. Biol. Chem. 266(25):16441 (1991); and Siegfried, J., Anat. Record 236: 241-247 (1993). Bombesin exhibits both direct and indirect effects on the gastrointestinal tract, including the release of hormones and the stimulation of pancreatic, gastric, and intestinal secretion and of intestinal mobility. GRP and cholecystokinin, which are released by bombesin, have been shown to play a role in the maintenance of normal gastrointestinal mucosa as well as in augmenting growth of normal and neoplastic tissues. The growth of xenografted human colon and stomach carcinomas in nude mice has been stimulated by the administration of gastrin and later inhibited with the addition of secretin (Tanake, et al., Tokaku, J. Exp. Med. 148:459, 1986) and the growth of MC-26 murine colon carcinoma which possesses gastrin receptors is stimulated by pentagastrin (Winsett, et al., Surgery 99:302 1980), and inhibited by proglumide, a gastrin-receptor antagonist (Beauchamp, et al., Ann. Surg. 202:303, 1985). Bombesin has been found to act concurrently as both a trophic agent for normal host pancreas and a growth inhibitory agent in xenografted human pancreatic tumor tissue. Alexander, et al., Pancreas 3:247, 1988. NMB has been shown to effect the growth of cancer cells (Moody, et al., J. Pharmacol. 261:1 (1992)), suppress food intake, and decrease gastrin release (Kawai, et al., Endocrinol. Japan 37(6):857 (1990)).

### SUMMARY OF THE INVENTION

Abbreviations:

Chx-Ala = cyclohexyl-Ala (3-cyclohexylalanine)
pGlu = pyroglutamic acid
Nle = norleucine
D-Cpa = D-p-chlorophenylalanine
HyPro = hydroxyproline
Nal = 3-(α-naphthyl)-alanine, or 3-(β-naphthyl)-alanine
DOPA = 3,4-dihydroxyphenylalanine
Tcc = 1, 2, 3, 4-tetrahydro-2-carboline-3-carboxylic acid
Tic = 1, 2, 3, 4-tetrahydroisoquinoline-3-carboxylic acid
Aza-Tyrosine = 3-(5-hydroxy-2-pyridyl)-alanine
Sar = sarcosine
1- or 3-methyl-His = His with a methyl group on its position 1 or 3 heterocyclic nitrogen
ε-alkyl-Lys = Lys with its $N^{\varepsilon}$ substituted by an alkyl group
β-Ala = 3-aminopropionic acid

The invention features linear therapeutic peptides of the following formula:

$$\begin{matrix} R_1 \\ R_2 \end{matrix} > A^1\text{-}A^2\text{-}A^3\text{-}A^4\text{-}A^5\text{-}A^6\text{-}A^7\text{-}A^8\text{-}A^9\text{-}R_3 \qquad \text{(I)}$$

in which:

$A^1$ is a D-α-aromatic amino acid or a D-α-tethered amino acid;
$A^2$ is Gln, His, 1-methyl-His, or 3-methyl-His;
$A^3$ is the D- or L-isomer selected from Nal, Trp, Phe, and p-X-Phe, where X is F, Cl, Br, $NO_2$, OH or $CH_3$;
$A^4$ is Ala, Val, Leu, Ile, Nle, or α-aminobutyric acid;
$A^5$ is Val, Ala, Leu, Ile, Nle, Thr, or α-aminobutyric acid;
$A^6$ is β-Ala;
$A^7$ is His, 1-methyl-His, 3-methyl-His, Lys, or ε-alkyl-Lys;
$A^8$ is Leu, Ile, Val, Nle, α-aminobutyric acid, Trp, Pro, Nal, Chx-Ala, Phe, or p-X-Phe, where X is F, Cl, Br, $NO_2$, OH or $CH_3$;
$A^9$ is Met, Met-oxide, Leu, Ile, Nle, α-aminobutyric acid, or Cys;
each $R_1$ and $R_2$, independently, is H, $C_{1-12}$ alkyl, $C_{7-10}$ phenylalkyl, or $COE_1$, where $E_1$ is $C_{1-20}$ alkyl, $C_{3-20}$ alkenyl, $C_{3-20}$ alkynyl, phenyl, 3,4-dihydroxyphenylalkyl, naphthyl, or $C_{7-10}$ phenylalkyl; provided that when either $R_1$ or $R_2$ is $COE_1$, the other must be H; and
$R_3$ is OH, $NH_2$, $C_{1-12}$ alkoxy, $C_{7-10}$ phenylalkoxy, $C_{11-20}$ naphthylalkoxy, $C_{1-12}$ alkylamino, $C_{7-10}$ phenylalkylamino, $C_{11-20}$ naphthylalkylamino; or a pharmaceutically acceptable salt of such peptides.

What is meant by "aromatic α-amino acid" is an amino acid residue of the formula $NH_2$-$CH(CH_2$-Z)-COOH where Z is a moiety containing an aromatic ring. Examples of Z include, but are not limited to, phenyl, 1-napthyl, 2-napthyl, 3-indolyl, 1-Me-3-indolyl, biphenyl, and imidazolyl, with or without one or more substituent X on the aromatic ring(s) of Z (where X is halogen, $NO_2$, $CH_3$, or OH).

What is meant by "tethered α-amino acid" is an α-amino acid with a carbon atom of its side chain tethered to the N atom of the α-amino group. Examples include, but are not limited to, Pro, HyPro, Tic, and Tcc.

The symbol $A^1$, $A^2$, or the like herein stands for the residue of an α-amino acid. Except for tethered amino acids (e.g., Pro, HyPro, Tcc, or Tic) and Sar, such symbols represent the general structure, -NH-CH(R)-CO- or =N-CH(R)-CO- when it is at the N-terminus or -NH-CH(R)-CO- when it is not at the N-terminus, where R denotes the side chain (or identifying group) of the α-amino acid, e.g., R is -$CH_2$COOH for Asp. Note that the N-terminus is at the left and the C-terminus at the right in accordance with the conventional representation of a polypeptide chain. When $A^6$ is Sar, it has the structure of -$N(CH_3)$-$CH_2$-CO-. The residue of a tethered amino acid, on the other hand, is of the structure -N-CH(R)-CO-, where N, C and R together form a ring. HyPro herein refers to any of 2-hydroxy-Pro, 3-hydroxy-Pro, 4-hydroxy-Pro, and 5-hydroxy-Pro; 4-hydroxy-Pro is preferred.

Furthermore, where the amino acid residue is optically active, it is the L-form configuration that is intended unless the D-form is expressly designated. An alkyl group, if not specified, contains 1-12 carbon atoms. $COE_1$ stands for

$$\overset{O}{C}\text{-}E_1$$

.

In formula (I) given above, when either of $R_1$ or $R_2$ is an aliphatic, aromatic, or lipophilic group, the in vivo activity can be long lasting, and delivery of the compounds of the invention to the target tissue can be facilitated.

Preferably, in formula (I), $A^1$ is the D-isomer selected from Nal, DOPA, Trp, Tcc, Tic, Aza-Tyr, Phe, and p-X-Phe, where X is F, Cl, Br, $NO_2$, OH or $CH_3$. It is particularly preferred that $A^3$ be the D-isomer selected from Phe, Trp, and p-X-Phe, where X is F, Cl, Br, $NO_2$, OH or $CH_3$; and $A^7$ be His, 1-methyl-His, or 3-methyl-His.

Also preferably, in formula (I), $A^1$ be D-Phe, D-Trp, or D-Tyr; $A^2$ be Gln; $A^3$ be Trp; $A^4$ be Ala; $A^5$ be Val; $A^7$ be His; $A^8$ be Leu or Phe; and $A^9$ be Met, Leu, and Nle.

Particularly preferred peptides of the invention include the following:

H-D-Phe-Gln-Trp-Ala-Val-β-Ala-His-Phe-Nle-$NH_2$;

H-D-Phe-Gln-Trp-Ala-Val-β-Ala-His-Leu-Leu-$NH_2$;
H-D-Tyr-Gln-Trp-Ala-Val-β-Ala-His-Phe-Nle-$NH_2$;
H-D-Trp-Gln-Trp-Ala-Val-β-Ala-His-Phe-Nle-$NH_2$; and
H-D-Phe-Gln-Trp-Ala-Val-β-Ala-His-Leu-Nle-$NH_2$.

Analogs of the invention can be provided in the form of pharmaceutically acceptable salts. Examples of preferred salts are those with therapeutically acceptable organic acids, e.g., acetic, lactic, maleic, citric, malic, ascorbic, succinic, benzoic, salicylic, methanesulfonic, toluene sulfonic, trifluoroacetic, or pamoic acid, as well as polymeric acids such as tannic acid or carboxymethyl cellulose, and salts with inorganic acids such as the hydrohalic acids, e.g., hydrochloric acid, sulfuric acid or phosphoric acid.

Other features and advantages of the invention will be apparent from the following description of the preferred embodiments thereof, and from the claims.

## BRIEF DESCRIPTION OF THE DRAWINGS

The drawings will be first described.

Fig. 1 is a graph showing the growth inhibitory effect of a compound of the present invention on prostate tumors.

## DESCRIPTION OF THE PREFERRED EMBODIMENTS

We now describe the structure, synthesis, biological assays, and use of the preferred embodiments of the present invention.

### Structure

Peptides of the invention are derived from litorin, neuromedin B (NMB), neuromedin C (NMC), bombesin (last ten amino acids), and human GRP (last ten amino acids).

### Synthesis of Analogs

The synthesis of one of the compounds of the invention, Analog #1 (i.e., H-D-Phe-Gln-Trp-Ala-Val-β-Ala-His-Phe-Nle-$NH_2$), follows.

4-methyl benzhydrylamine-polystyrene resin (Bachem, Inc.) (0.72 meq/g), in the chloride ion form, was placed in the reaction vessel of an ACT200 peptide synthesizer (Advanced Chem Tech, Inc.) programmed to perform the following reaction cycle: (a) methylene chloride; (b) 10% triethylamine in chloroform; (c) methylene chloride; and (d) dimethylformide. The neutralized resin was mixed with BOC-Norleucine and diisopropylcarbodiimide (3 molar eq each) in methylene chloride for 1 hour. The resulting amino acid resin was washed on the synthesizer with dimethylformamide and treated with 5% acetic anhydride in dimethylformamide for 5 min. Then it was washed with dimethylformamide and methylene chloride.

The peptide synthesizer was programmed to perform the following reaction cycle: (a) methylene chloride; (b) 33% trifluoroacetic acid ("TFA") in methylene chloride (2 times for 5 and 25 min. each); (c) methylene chloride; (d) isopropyl alcohol; (e) 10% triethylamine in chloroform; and (f) methylene chloride.

The following amino acids (3 molar eq.) are then coupled successively by the same procedure: BOC-Phe, BOC-His (CBZ), BOC-β-Ala, BOC-Val, BOC-Ala, BOC-Trp, BOC-Gln (coupled in the presence of 1 eq. hydroxybenzotriazole), and BOC-D-Phe (coupled in the presence of 1 eq. hydroxybenzotriazole). The completed resin was then washed with methanol and air dried.

The peptide resin described above (3.5076 g) was mixed with anisole (10 ml), dithiothreitol (100 mg), and anhydrous hydrogen fluoride (50 ml) at 0°c for one hour. Excess hydrogen fluoride was evaporated rapidly under a stream of dry nitrogen, and the residue was washed in ether. Crude peptide was dissolved in 100 ml of 4 M acetic acid and the solution is then evaporated under reduced pressure. The crude peptide was dissolved in minimum volume of methanol/water and triturated with ethyl acetate. The triturated peptide was applied to a column (9.4 mm I.D. x 50 cm) of octadecylcilane-silica (Whatman Partisil 10 ODS - 2M9). The peptide was eluted with a linear gradient of 20-80% of 50/50 0.1% TFA/Acetronitrile i 0.1% TFA in water. Fractions were examined by analytical high performance liquid chromatography and appropriate fractions were evaporated to a small volume, which was further lyophilized, giving 80 mg of the product as a colorless powder.

Other compounds of the present invention, e.g., Analog #2 (i.e., H-D-Phe-Gln-Trp-Ala-Val-β-Ala-His-Leu-Leu-$NH_2$), can be prepared in an analogous manner by making appropriate modifications of the above-described synthetic method.

Biological Assays

(1) GRP Receptor Binding Assay

Rat AR42J pancreatic acinar cells were cultured in Dulbecco's modified Eagle's medium without antibiotics and supplemented with 10% (vol/vol) fetal calf serum. The incubation atmosphere consisted of 10% $CO_2$-90% humidified air at 37°C.

Membranes for the bombesin receptor binding assay were obtained by homogenizing AR42J cells (Polytron, setting 6, 15 sec)(Brinkman, Westbury, NY) in ice-cold 50 mM Tris-HCl (Buffer A) and centrifuging twice at 39,000 x g (10 min), with an intermediate resuspension in fresh Buffer A. The final pellets were resuspended in 50 mM Tris-HCl, containing 0.1 mg/ml bacitracin, and 0.1% BSA (Buffer B), and held on ice for the receptor binding assay.

For assay, aliquots (0.4 ml) were incubated with 0.05 ml [$^{125}$ I-Tyr$^4$] bombesin (~2200 Ci/mmol, New England Nuclear) and Buffer B, with and without 0.05 ml of unlabeled competing analogs. After a 30 min incubation (4°C), the bound [$^{125}$ I-Tyr$^4$] bombesin was separated from the free by rapid filtration through Whatman™ GF/B filters which had been previously soaked in 0.1% polyethyleneimine using a Brandel filtration manifold. The filters were then washed three times with 5 ml aliquots of ice-cold Buffer A. Specific binding was defined as the total [$^{125}$ I-Tyr$^4$] bombesin bound minus that bound in the presence of 1 μM unlabeled bombesin.

The results (expressed as $IC^{50}$ in nM) and the structures of the tested compounds are shown in Table 1. Replacement of Gly at position $A^6$ in a prior art compound (i.e., litorin, Leu-litorin, and [D-Phe$^1$, Leu$^{8,9}$] litorin) with β-Ala led unexpectedly to a great increase in their affinity for the GRP receptor. For example, replacement of Gly with β-Ala resulted in a 2.8 to 23 time increase in the affinity (compare Analog #2 with [D-Phe$^1$, Leu$^{8,9}$] litorin or Leu-litorin). Modifications at position $A^1$, $A^8$, or $A^9$ further increases the affinity. For example, Analog #3 has an $IC_{50}$ being as low as 0.03 nM, compared to 2.4 nM for litorin, 23 nM for Leu-litorin, or 2.8 nM for [D-Phe$^1$, Leu$^{8,9}$] litorin-- an increase of 80 to 766 times in the affinity for the GRP receptor.

TABLE 1

AFFINITY BINDING DATA FOR THE GRP RECEPTOR OF LINEAR BOMBESIN ANALOGS WITH β-Ala REPLACEMENT AT POSITION $A^{6*}$

| Code Name | Structure | $IC_{50}$ (nM) |
|---|---|---|
| Litorin | p-Glu-Gln-Trp-Ala-Val-Gly-His-Phe-Met-$NH_2$ | 2.4 |
| Leu-Litorin | p-Glu-Gln-Trp-Ala-Val-Gly-His-Leu-Leu-$NH_2$ | 23 |
| [D-Phe$^1$, Leu$^{8,9}$] litorin | H-D-Phe-Gln-Trp-Ala-Val-Gly-His-Leu-Leu-$NH_2$ | 2.8 |
| Analog #1 | H-D-Phe-Gln-Trp-Ala-Val-β-Ala-His-Phe-Nle-$NH_2$ | 0.2 |
| Analog #2 | H-D-Phe-Gln-Trp-Ala-Val-β-Ala-His-Leu-Leu-$NH_2$ | 1.0 |
| Analog #3 | H-D-Tyr-Gln-Trp-Ala-Val-β-Ala-His-Phe-Nle-$NH_2$ | 0.03 |
| Analog #4 | H-D-Trp-Gln-Trp-Ala-Val-β-Ala-His-Phe-Nle-$NH_2$ | 0.1 |
| Analog #5 | H-D-Phe-Gln-Trp-Ala-Val-β-Ala-His-Leu-Nle-$NH_2$ | 2.1 |

* See formula (I) in "SUMMARY OF THE INVENTION" above.

(2) NMB Receptor Binding Assay

The procedure for transfecting the rat NMB receptor into BALB-3T3 fibroblasts is discussed in Wada, et al., *Neuron*,

6:4221-430 (1991) and Benya, et al., *Mol. Pharmacol.*, 42:1058 (1992).

Membranes for the bombesin receptor binding assay were obtained by homogenizing BALB-373 fibroblasts, transfected with the rat NMB receptor (Polytron, setting 6, 15 sec) in ice-cold 50 mM Tris-HCl (Buffer A) and centrifuging twice at 39,000 x g (10 min), with an intermediate resuspension in fresh Buffer A. The final pellets were resuspended in 50 mM Tris-HCl, containing 0.1 mg/ml bacitracin, and 0.1% BSA (Buffer B), and held on ice for the receptor binding assay.

For assay, aliquots (0.4 ml) were incubated with 0.05 ml [$^{125}$ I-Tyr$^4$] bombesin (~2200 Ci/mmol, New England Nuclear) and Buffer B, with and without 0.05 ml of unlabeled competing analogs. After a 30 min incubation (4°C), the bound [$^{125}$ I-Tyr$^4$] bombesin was separated from the free by rapid filtration through Whatman™ GF/B filters which had been previously soaked in 0.3% polyethyleneimine using a Brandel filtration manifold. The filters were then washed three times with 5 ml aliquots of ice-cold Buffer A. Specific binding was defined as the total [$^{125}$ I-Tyr$^4$] neuromedin-B bound minus that bound in the presence of 1 μM unlabeled neuromedin-B.

The results (expressed as IC$^{50}$ in nM) and the structures of the tested compounds are shown in Table 1. Replacement of Gly at position A$^6$ in a prior art compound (i.e., litorin, leu-litorin, and [D-Phe$^1$, Leu$^{8,9}$] litorin) with β-Ala led unexpectedly to a great increase in their affinity for the NMB receptor. For example, replacement of Gly with β-Ala resulted in a 11.7 time increase in the affinity (compare Analog #2 with [D-Phe$^1$, Leu$^{8,9}$] litorin). Modifications at position A$^1$, A$^8$, or A$^9$ further increases the affinity. For example, Analog #4 has an IC$_{50}$ being as low as 0.04 nM, compared to 1.66 nM for litorin or 480 nM for [D-Phe$^1$, Leu$^{8,9}$] litorin-- an increase of 41.5 to 12,000 times in the affinity for the NMB receptor. Leu-litorin was found to have almost no binding affinity on the NMB receptor.

TABLE 2

AFFINITY BINDING DATA FOR THE NMB RECEPTOR OF LINEAR BOMBESIN ANALOGS WITH β-Ala REPLACEMENT AT POSITION A$^{6*}$

| Code Name | Structure | IC$_{50}$ (nM) |
|---|---|---|
| Litorin | p-Glu-Gln-Trp-Ala-Val-Gly-His-Phe-Met-$NH_2$ | 1.7 |
| Leu-Litorin | p-Glu-Gln-Trp-Ala-Val-Gly-His-Leu-Leu-$NH_2$ | >10,000 |
| [D-Phe$^1$, Leu$^{8,9}$] litorin | H-D-Phe-Gln-Trp-Ala-Val-Gly-His-Leu-Leu-$NH_2$ | 480 |
| Analog #1 | H-D-Phe-Gln-Trp-Ala-Val-β-Ala-His-Phe-Nle-$NH_2$ | 1.0 |
| Analog #2 | H-D-Phe-Gln-Trp-Ala-Val-β-Ala-His-Leu-Leu-$NH_2$ | 41 |
| Analog #3 | H-D-Tyr-Gln-Trp-Ala-Val-β-Ala-His-Phe-Nle-$NH_2$ | 1.0 |
| Analog #4 | H-D-Trp-Gln-Trp-Ala-Val-β-Ala-His-Phe-Nle-$NH_2$ | 0.04 |
| Analog #5 | H-D-Phe-Gln-Trp-Ala-Val-β-Ala-His-Leu-Nle-$NH_2$ | 8.9 |

* See formula (I) in "SUMMARY OF THE INVENTION" above.

(3) Assay on in vivo prostate tumor growth

The androgen responsive R-3327/H prostate tumor line was implanted into 38 testes-intact, syngeneic Copenhagen male rats. The tumored animals were individually staged for castration when their tumors reached a size approximating 235 mgs. Tumors were then staged for bombesin analogue treatment when two sequential tumor measurements indicated an "escape from castration inhibition" condition, as evidenced by an increase in tumor growth rate. This transition of the prostate tumor tissue from androgen sensitive to androgen insensitive occurred without surgical perturbation of the tumor, thus providing a clinically realistic model for evaluating the anti-prostate tumor activity of the bombesin analogues.

The tumored animals were separated into two groups of 19 animals. Treatment was only initiated when tumors reached the "escape from castration inhibition" condition. Group 1 received 0.2 ml/inj. of 2.6% glycerol/water vehicle, (s.c., B.I.D.). Group 2 received a 200 mg/inj. of Analog #5 (s.c., B.I.D.). Both injections were administered in the flank opposite from the tumor. The treatment period lasted for 24 days. Tumor measurements were taken at day 3, day 7, day 10, day 14, day 17, day 21, and day 24. Tumors were measured using Vernier calipers and the volume was calculated using the following formula: $(0.5)(\text{length})(\text{width})^2$

Fig. 1 illustrates the tumor growth inhibitory effect of the treatments in Group 1 and Group 2. Relative tumor volume was calculated by the following formula:

$$\frac{\text{Tumor Volume (measuring day)}}{\text{Tumor Volume (staging day)}} \times 100$$

The data is provided by the mean relative tumor volume ± standard error. There was a steadily increasing divergence in the size of vehicle treated control tumors (Group 1) and tumors treated with Analog #5 (Group 2) during the first 21 days of treatment. Rate of tumor growth was significantly ($p < 0.05$) inhibited by Analog #5 treatment after day 17.

Use

Analogs of the invention are useful for treating colon, prostatic, breast, pancreatic, liver cancer or lung cancer, for preventing the proliferation of smooth muscle, for suppressing appetite, for stimulating pancreatic secretion, or for suppressing a craving for alcohol. Analogs of the invention are administered to a mammal, particularly a human, in one of the traditional modes (e.g., orally, parenterally, transdermally, transmucosally, or via drug-releasing implants), in a sustained release formulation using a biodegradable biocompatible polymer, or by on-site delivery using micelles, gels and liposomes, or rectally (e.g., by suppository or enema). The analogs can be administered to a human patient in a dosage to be determined by the attending physician ranging from 0.25 mg/kg/day to 5 mg/kg/day.

Furthermore, compounds of the present invention, particularly those with Tyr at the N-terminus, can be used for diagnostic purposes and for the tumor targeting of radioisotopes such as $^{131}$Iodine.

OTHER EMBODIMENTS

The foregoing description has been limited to specific embodiments of this invention. It will be apparent, however, that variations and modifications may be made to the invention, with the attainment of some or all of the advantages of the invention. Such embodiments are also within the scope of the following claims.

## Claims

**1.** A peptide of the formula:

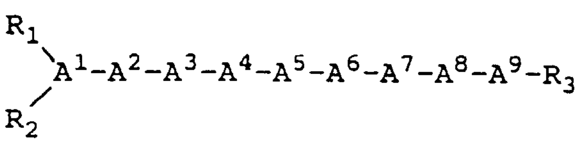

wherein:

$A^1$ is a D-α-aromatic amino acid or a D-α-tethered amino acid;
$A^2$ is Gln, His, 1-methyl-His, or 3-methyl-His;
$A^3$ is the D- or L-isomer selected from Nal, Trp, Phe, and p-X-Phe, where X is F, Cl, Br, $NO_2$, OH or $CH_3$;
$A^4$ is Ala, Val, Leu, Ile, Nle, or α-aminobutyric acid;
$A^5$ is Val, Ala, Leu, Ile, Nle, Thr, or α-aminobutyric acid;
$A^6$ is β-Ala;
$A^7$ is His, 1-methyl-His, 3-methyl-His, Lys, or ε-alkyl-Lys;

$A^8$ is Leu, Ile, Val, Nle, $\alpha$-aminobutyric acid, Trp, Pro, HyPro, Nal, Chx-Ala, Phe, or p-X-Phe, where X is F, Cl, Br, $NO_2$, OH or $CH_3$;
$A^9$ is Met, Met-oxide, Leu, Ile, Nle, $\alpha$-aminobutyric acid, or Cys;
each $R_1$ and $R_2$, independently, is H, $C_{1-12}$ alkyl, $C_{7-10}$ phenylalkyl, or $COE_1$, where $E_1$ is $C_{1-20}$ alkyl, $C_{3-20}$ alkenyl, $C_{3-20}$ alkynyl, phenyl, 3,4-dihydroxyphenylalkyl, naphthyl, or $C_{7-10}$ phenylalkyl; provided that when either $R_1$ or $R_2$ is $COE_1$, the other must be H; and
$R_3$ is OH, $NH_2$, $C_{1-12}$ alkoxy, $C_{7-10}$ phenylalkoxy, $C_{11-20}$ naphthylalkoxy, $C_{1-12}$ alkylamino, $C_{7-10}$ phenylalkylamino, $C_{11-20}$ naphthylalkylamino; or a pharmaceutically acceptable salt thereof.

2. The peptide of claim 1, wherein $A^1$ is the D-isomer selected from Nal, DOPA, Trp, Tcc, Tic, Aza-Tyr, Phe, and p-X-Phe, where X is F, Cl, Br, $NO_2$, OH or $CH_3$.

3. The peptide of claim 2, wherein $A^3$ is the D-isomer selected from Trp, Phe, and p-X-Phe, where X is F, Cl, Br, $NO_2$, OH or $CH_3$; and $A^7$ is His, 1-methyl-His, or 3-methyl-His.

4. The peptide of claim 3, wherein $A^3$ is Trp and/or $A^7$ is His and/or $A^2$ is Gln and/or $A^4$ is Ala and/or $A^5$ is Val and/or $A^8$ is Leu or Phe and/or $A^9$ is Met, Leu or Nle and/or $A^1$ is D-Phe, D-Tyr or D-Trp.

5. The therapeutic peptide of claim 4 of the formula:

H-D-Phe-Gln-Trp-Ala-Val-β-Ala-His-Phe-Nle-$NH_2$

or

H-D-Phe-Gln-Trp-Ala-Val-β-Ala-His-Leu-Leu-$NH_2$

or

H-D-Tyr-Gln-Trp-Ala-Val-β-Ala-His-Phe-Nle-$NH_2$

or

H-D-Trp-Gln-Trp-Ala-Val-β-Ala-His-Phe-Nle-$NH_2$

or

H-D-Phe-Gln-Trp-Ala-Val-β-Ala-His-Leu-Nle-$NH_2$

6. The peptide of any one of claims 1 to 5 for use in a method of medical treatment.

7. Use of a peptide according to any one of claims 1 to 5 in the manufacture of a medicament for the treatment of colon, prostatic, breast, pancreatic, liver or lung cancer, for preventing the proliferation of smooth muscle, for suppressing appetite, for stimulating pancreatic secretion, or for suppressing a craving for alcohol.

**Patentansprüche**

1. Peptid der Formel:

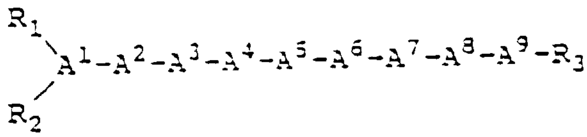

worin

$A^1$ eine aromatische D-α-Aminosäure oder eine D-α-ringbildende-Aminosäure ist;
$A^2$ Gln, His, 1-Methyl-His oder 3-Methyl-His ist;
$A^3$ das aus Nal, Trp, Phe und p-X-Phe ausgewählte D- oder L-Isomer ist, worin X = F, Cl, Br, $NO_2$, OH oder $CH_3$ ist;
$A^4$ Ala, Val, Leu, Ile, Nle oder α-Aminobuttersäure ist;
$A^5$ Val, Ala Leu, Ile, Nle, Thr oder α-Aminobuttersäure ist;
$A^6$ β-Ala ist;
$A^7$ His, 1-Methyl-His, 3-Methyl-His, Lys oder ε-Alkyl-Lys ist;
$A^8$ Leu, Ile, Val, Nle, α-Aminobuttersäure, Trp, Pro, HyPro, Nal, Chx-Ala, Phe oder p-X-Phe ist, worin X = F, Cl, Br, $NO_2$, OH oder $CH_3$ ist;
$A^9$ Met, Met-Oxid, Leu, Ile, Nle, α-Aminobuttersäure oder Cys ist;
jedes $R_1$ und $R_2$, unabhängig voneinander, H, $C_{1-12}$-Alkyl, $C_{7-10}$-Phenylalkyl oder $COE_1$ ist, worin $E_1$ = $C_{1-20}$-Alkyl, $C_{3-20}$-Alkenyl, $C_{3-20}$-Alkinyl, Phenyl, 3,4-Dihydroxyphenylakyl, Naphthyl oder $C_{7-10}$-Phenylalkyl ist; mit der Maßgabe, daß, wenn entweder $R_1$ oder $R_2$ = $COE_1$ ist, das andere H sein muß, und
$R_3$ = OH, $NH_2$, $C_{1-12}$-Alkoxy, $C_{7-10}$-Phenylalkoxy, $C_{11-20}$-Naphthylalkoxy, $C_{1-12}$-Alkylamino, $C_{7-10}$-Phenylalkylamino, $C_{11-20}$-Naphthylalkylamino ist; oder ein pharmazeutisch annehmbares Salz davon.

**2.** Peptid nach Anspruch 1, worin $A^1$ das aus Nal, DOPA, Trp, Tcc, Tic, Aza-Tyr, Phe und p-X-Phe ausgewählte D-Isomer ist, worin X = F, Cl, Br, $NO_2$, OH oder $CH_3$ ist.

**3.** Peptid nach Anspruch 2, worin $A^3$ das aus Trp, Phe und p-X-Phe ausgewählte D-Isomer ist, worin X = F, Cl, Br, $NO_2$, OH oder $CH_3$ ist; und $A^7$ = His 1-Methyl-His oder 3-Methyl-His ist.

**4.** Peptid nach Anspruch 3, worin $A^3$ = Trp ist und/oder $A^7$ = His ist und/oder $A^2$ = Gln ist und/oder $A^4$ = Ala ist und/oder $A^5$ = Val ist und/oder $A^8$ = Leu oder Phe ist und/oder $A^9$ = Met, Leu oder Nle ist und/oder $A^1$ = D-Phe D-Tyr oder D-Trp ist.

**5.** Peptid nach Anspruch 4 der Formel:

H-D-Phe-Gln-Trp-Ala-Val-β-Ala-His-Phe-Nle-$NH_2$

oder

H-D-Phe-Gln-Trp-Ala-Val-β-Ala-His-Leu-Leu-$NH_2$

oder

H-D-Tyr-Gln-Trp-Ala-Val-β-Ala-His-Phe-Nle-$NH_2$

oder

H-D-Trp-Gln-Trp-Ala-Val-β-Ala-His-Phe-Nle-$NH_2$

oder

H-D-Phe-Gln-Trp-Ala-Val-β-Ala-His-Leu-Nle-$NH_2$.

6. Peptid nach einem der Ansprüche 1 bis 5 zur Verwendung bei einem Verfahren zur medizinischen Behandlung.

7. Verwendung eines Peptids nach einem der Ansprüche 1 bis 5 bei der Herstellung eines Medikaments zur Behandlung von Grimmdarm-, Prostata-, Brust-, Bauchspeicheldrüsen-, Leber- oder Lungenkrebs, zur Verhinderung des Wachstums von glattem Muskel, zur Appetitunterdrückung, zum Stimulieren der Pankreassekretion oder zum Unterdrücken eines Verlangens nach Alkohol.

**Revendications**

1. Peptide de formule :

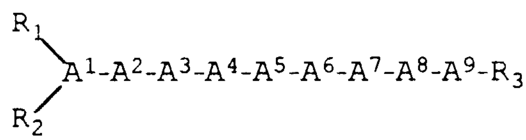

où

$A^1$ est un acide aminé D-α-aromatique ou un acide aminé D-α-attaché;

$A^2$ est Gln, His, 1-méthyl-His, ou 3-méthyl-His;

$A^3$ est l'isomère D- ou L- sélectionné parmi Nal, Trp, Phe, et p-X-Phe, où X est F, Cl, Br, $NO_2$, OH ou $CH_3$;

$A^4$ est Ala, Val, Leu, Ile, Nle, ou l'acide α-aminobutyrique;

$A^5$ est Val, Ala, Leu, Ile, Nle, Thr, ou l'acide α-aminobutyrique;

$A^6$ est β-Ala;

$A^7$ est His, 1-méthyl-His, 3-méthyl-His, Lys, ou ε-alkyl-Lys;

$A^8$ est Leu, Ile, Val, Nle, l'acide α-aminobutyrique, Trp, Pro, HyPro, Nal, Chx-Ala, Phe, ou p-X-Phe, où X est F, Cl, Br, $NO_2$, OH ou $CH_3$;

$A^9$ est Met, Met-oxyde, Leu, Ile, Nle, l'acide α-aminobutyrique, ou Cys;

chacun de $R^1$ et $R^2$, indépendamment, est H, un alkyle en $C_{1-12}$, un phénylalkyle en $C_{7-10}$, ou $COE_1$, où $E_1$ est un alkyle en $C_{1-20}$, un alkényle en $C_{3-20}$, un alcynyle en $C_{3-20}$, un phényle, un 3,4-dihydroxyphénylalkyle, un naphthyle, ou un phénylalkyle en $C_{7-10}$; à condition que lorsque soit $R_1$ soit $R_2$ est $COE_1$, l'autre doit être H; et

$R_3$ est OH, $NH_2$, un alcoxy en $C_{1-12}$, un phénylalcoxy en $C_{7-10}$, un naphthylalcoxy en $C_{11-20}$, un alkylamino en $C_{1-12}$, un phénylalkylamino en $C_{7-10}$, un naphthylalkylamino en $C_{11-20}$; ou un sel pharmaceutiquement acceptable de ceux-ci.

2. Peptide selon la revendication 1, dans lequel $A^1$ est l'isomère-D sélectionné parmi Nal, DOPA, Trp, Tcc, Tic, Aza-Tyr, Phe, et p-X-Phe, où X est F, Cl, Br, $NO_2$, OH ou $CH_3$.

3. Peptide selon la revendication 2, où $A^3$ est l'isomère-D sélectionné parmi Trp, Phe, et p-X-Phe, où X est F, Cl, Br, $NO_2$, OH ou $CH_3$; et $A^7$ est His, 1-méthyl-His, ou 3-méthyl-His.

**4.** Peptide selon la revendication 3, où $A^3$ est Trp et/ou $A^7$ est His et/ou $A^2$ est Gln et/ou $A^4$ est Ala et/ou $A^5$ est Val et/ou $A^8$ est Leu ou Phe et/ou $A^9$ est Met, Leu ou Nle et/ou $A^1$ est D-Phe, D-Tyr ou D-Trp.

**5.** Peptide thérapeutique selon la revendication 4 de formule :

$$H\text{-}D\text{-}Phe\text{-}Gln\text{-}Trp\text{-}Ala\text{-}Val\text{-}\beta\text{-}Ala\text{-}His\text{-}Phe\text{-}Nle\text{-}NH_2$$

ou

$$H\text{-}D\text{-}Phe\text{-}Gln\text{-}Trp\text{-}Ala\text{-}Val\text{-}\beta\text{-}Ala\text{-}His\text{-}Leu\text{-}Leu\text{-}NH_2$$

ou

$$H\text{-}D\text{-}Tyr\text{-}Gln\text{-}Trp\text{-}Ala\text{-}Val\text{-}\beta\text{-}Ala\text{-}His\text{-}Phe\text{-}Nle\text{-}NH_2$$

ou

$$H\text{-}D\text{-}Trp\text{-}Gln\text{-}Trp\text{-}Ala\text{-}Val\text{-}\beta\text{-}Ala\text{-}His\text{-}Phe\text{-}Nle\text{-}NH_2$$

ou

$$H\text{-}D\text{-}Phe\text{-}Gln\text{-}Trp\text{-}Ala\text{-}Val\text{-}\beta\text{-}Ala\text{-}His\text{-}Leu\text{-}Nle\text{-}NH_2$$

**6.** Peptide selon l'une quelconque des revendications 1 à 5 pour utilisation dans une méthode de traitement médical.

**7.** Utilisation d'un peptide selon l'une quelconque des revendications 1 à 5 dans la fabrication d'un médicament pour le traitement du cancer du colon, de la prostate, du sein, du pancréas, du foie ou du poumon, pour prévenir la prolifération du muscle lisse, pour supprimer l'appétit, pour stimuler la sécrétion pancréatique, ou pour supprimer un désir impérieux de consommation d'alcool.

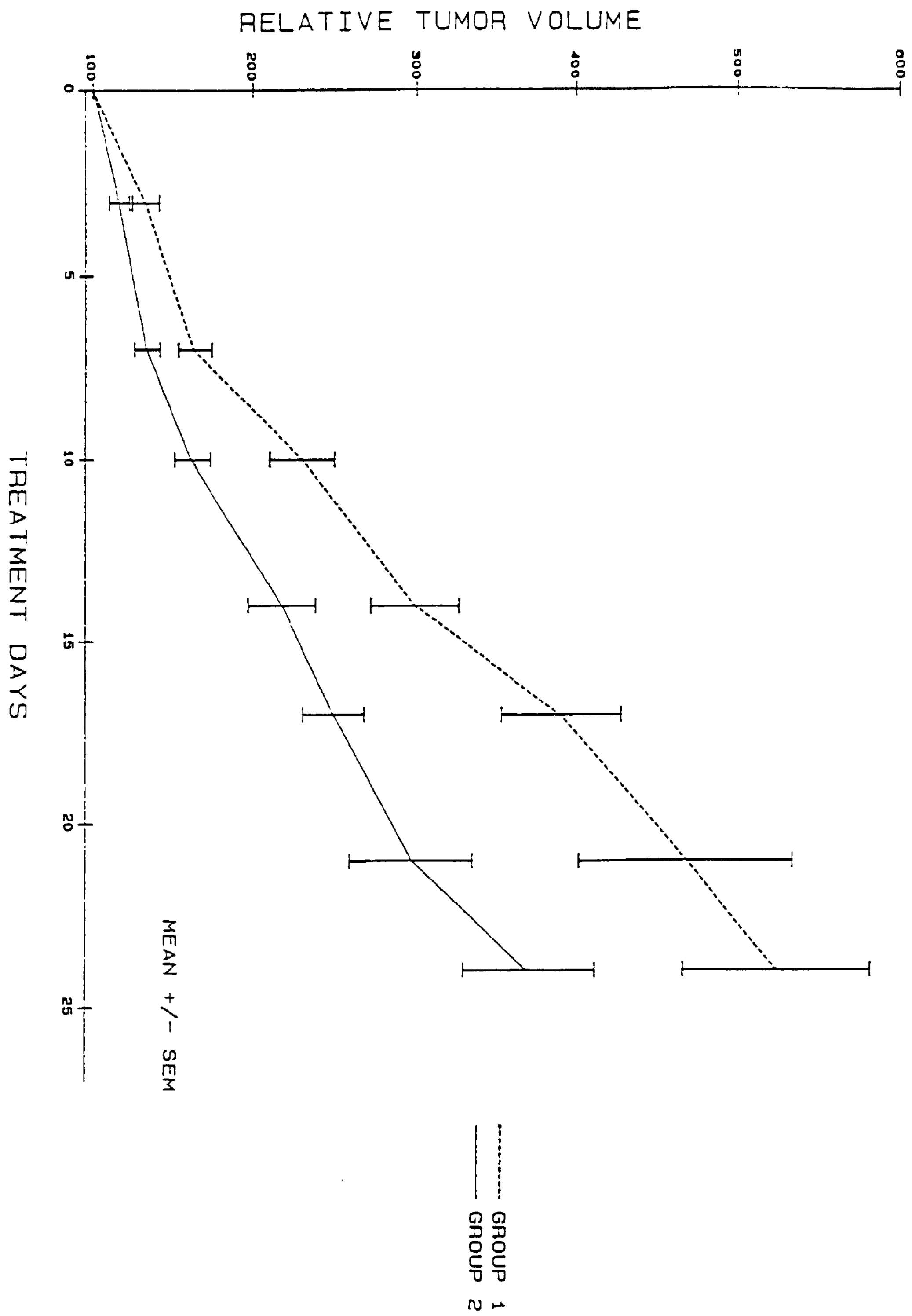
RELATIVE TUMOR VOLUME
100
200
300
400
500
600
0
5
10
15
20
25
TREATMENT DAYS
MEAN +/- SEM
GROUP 1
GROUP 2

Fig. 1